# EUROPEAN PATENT APPLICATION

(11) **EP 3 991 786 A1**
(43) Date of publication of application: **04.05.2022**
(21) Application number: 21205359.9
(22) Date of filing: 28.10.2021
(51) Int. Cl.: A61N 1/36

(54) **SLEEP APNEA THERAPY**

(30) Priority: 30.10.2020 US 202063107717 P; 07.10.2021 US 202117450271
(71) Applicant: Medtronic Xomed, Inc., Jacksonville, FL 32216-0980 (US)
(72) Inventor: SCHEINER, Avram, Vadnais Heights, MN (US); SCHULHAUSER, Randal C., Phoenix, AZ (US)
(74) Representative: Dehns

(57) **Abstract**

A system for sleep apnea treatment includes an implantable medical device (IMD) (30) coupled to a first lead (40) and a second lead (44), wherein the IMD comprises a processor (50) and stimulation circuitry (55), and wherein the processor is configured to cause the stimulation circuitry of IMD to: transmit a first stimulation signal to the first lead to stimulate at least one of an ansa cervicalis, a glossopharyngeal nerve, tensor veli, levator veli, and digastric anterior of a patient, and transmit a second stimulation signal to the second lead to stimulate at least one a hypoglossal nerve or a phrenic nerve of the patient.

## Description

This application claims the benefit of U.S. Provisional Patent Application No. 63/107,717, filed on October 30, 2020.

### TECHNICAL FIELD

Disclosed is a system to apply therapy to a subject for sleep apnea.

### BACKGROUND

A subject may have a condition that may be harmful to the subject, including over a period of time. One condition may include obstruction of an airway for an air-breathing subject, such as a human. Several conditions exist that may obstruct an airway.

Obstructive sleep apnea (OSA), central sleep apnea (CSA), and upper airway restrictive/resistance syndrome (UARS) are examples of sleep apneas that obstruct the airway, and cause lack of adequate levels of oxygen during sleep. Lack of adequate levels of oxygen during sleep can contribute to abnormal heart rhythms, heart attack, heart failure, high blood pressure, stroke, memory problems, and increased accidents during the day due to inadequate sleep. Additionally, loss of sleep occurs when a person is awakened during an apneic episode.

### SUMMARY

Conditions that may affect a subject breathing may include Sleep Apnea (SA) where the subject ceases breathing during sleep. There are several types of SA such as obstructive sleep apnea (OSA) and/or upper airway restrictive/resistance syndrome (UARS). One difference between OSA and UARS may be that apneas (e.g., pauses in breathing) or hypopneas (e.g., decreases in breathing) may be present in a patient with OSA, and may be absent or low in patient with UARS. OSA and UARS may be caused by a collapse or relaxing of various muscles and/or muscle groups in the upper airway that may be described as medial/lateral and/or anterior/posterior. Anterior/posterior collapse may include collapse of the genioglossus which may be treated with stimulation of this muscle and/or the hypoglossal nerve. Medial/lateral collapse may occur when one of more of the sternohyoid, sternothyroid, and omohyoid muscles relaxes or the airway collapses (e.g., the muscle relaxing may cause the airway to collapse). Concentric collapse may refer to an occurrence when both type of collapse occur simultaneously. OSA and UARS may affect a subject by limiting airflow and, therefore, oxygen saturation. Low oxygen saturation may lead to various further undesired conditions. Treatment of these conditions often requires an external device to assist in providing airflow to a subject.

Treatment of OSA and/or UARS may be provided by stimulating a selected portion of a subject. The stimulation may be provided by an implanted device. Thus, the treatment may be provided without an externally worn or applied system. Treatment, therefore, may be automatic and less or non-obtrusive.

In various examples, a stimulation system may be provided to a subject to stimulate various portions of the subject. The stimulation system may include a system that is able to provide an electrical stimulation, or other appropriate stimulation, from a source to a subject, such as through a lead. The lead may include one or more contacts or electrodes to provide the stimulation from the source to the selected area. The source may include a generator and/or a power source to provide the stimulation to a selected area.

To treat OSA and/or UARS the stimulation may be to a portion of a lingual muscle. The stimulation may be provided through the lead from an implanted stimulator according to at least one waveform and/or configuration. Further, the system may include a learning and/or testing phase to select one or more patterns or configurations. The selected patterns may be predetermined or determined in real time based on feedback from selected one or more sensors and/or individuals (e.g., subject, clinician, etc.)

In various examples, the stimulation may be provided to a human subject or an animal subject to stimulate a selected portion of the subject. The stimulation may include the electrical stimulation to cause a selected muscle to activate and stiffen or contract. Contraction of a muscle, as is generally understood by one skilled in the art, may cause a muscle to contract and shorten to cause movement of a selected subject portion, such as an appendage, a muscle tissue portion, or other selected portion of the subject.

Additionally, SA may also be caused by central sleep apnea (CSA). During CSA, the subject's diaphragm may not expand and contract to cause breathing. Different from OSA, CSA is not caused by an obstruction of the breathing pathway, but a lack of stimulation and/or activation of muscles that cause breathing. CSA, therefore, often requires a different or separate treatment than OSA.

A subject may also have both OSA and CSA. Inclusive or complicated SA (ISA) may require complex stimulation. The stimulation may be that of several sites in various manners. Stimulation of the several sites may be sequential, serial, simultaneous, or other appropriate manner.

A single stimulation device with a plurality of channels is disclosed that includes the possibility of stimulating a plurality of locations simultaneously, sequentially, and/or selectively. The multiple locations may be selectively stimulated based on sensed condition of the subject and/or instructions provided to the stimulation device. The stimulation device may be implanted and/or external to the subject.

In one example, the disclosure describes a system for sleep apnea treatment, comprising: an implantable medical device (IMD) couplable to a first lead and a second lead, wherein the IMD comprises a processor and stimulation circuitry, and wherein the processor is configured to cause the stimulation circuitry of the IMD to: transmit a first stimulation signal to the first lead to stimulate at least one of an ansa cervicalis, a glossopharyngeal nerve, tensor veli, levator veli, and digastric anterior of a patient; and transmit a second stimulation signal to the second lead to stimulate at least one a hypoglossal nerve or a phrenic nerve of the patient.

In one example, the disclosure describes a method for sleep apnea treatment, the method comprising: transmitting, with an implantable medical device (IMD), a first stimulation signal to a first lead to stimulate at least one of an ansa cervicalis, a glossopharyngeal nerve, tensor veli, levator veli, and digastric anterior of a patient; and transmitting, with the same IMD, a second stimulation signal to the second lead to stimulate at least one a hypoglossal nerve or a phrenic nerve of the patient.

In one example, the disclosure describes a computer-readable storage medium storing instructions thereon that when executed cause one or more processors of an implantable medical device (IMD) to: cause the IMD to transmit a first stimulation signal to a first lead to stimulate at least one of an ansa cervicalis, a glossopharyngeal nerve, tensor veli, levator veli, and digastric anterior of a patient; and cause the same IMD to transmit a second stimulation signal to the second lead to stimulate at least one a hypoglossal nerve or a phrenic nerve of the patient.

Further areas of applicability will become apparent from the description provided herein. The description and specific examples in this summary are intended for purposes of illustration only and are not intended to limit the scope of the present disclosure.

### BRIEF DESCRIPTION OF DRAWINGS

FIG. 1 is a schematic illustration of a stimulation system.
FIG. 2 is a schematic illustration of an implantation position of a stimulation system.
FIG. 3A, 3B, and 3C illustrate stimulations patterns, according to various examples.
FIG. 4 is a flowchart of a process for treating one or more types of sleep apnea, according to various embodiments.
FIG. 5 is a flowchart illustrating an example method of sleep apnea treatment.

Corresponding reference numerals indicate corresponding parts throughout the several views of the drawings.

### DETAILED DESCRIPTION

The devices, systems, and techniques of this disclosure generally relate to a single implantable medical device (IMD) for delivering therapy to address different mechanisms of sleep apnea. For instance, this disclosure describes examples of integration of stimulation of various tongue, upper airway, and/or neck muscles to produce coordinated support for upper airway opening during sleep.

By integrating stimulation of various muscles with a single IMD, the example techniques may improve overall treatment of sleep apnea, while minimizing implantable burden. For example, obstructive sleep apnea (OSA) is one mechanism for sleep apnea, and with hypoglossal nerve stimulation, the tongue can be caused to move forward, and open the airway. However, hypoglossal nerve stimulation may not be fully effective for patients with concentric collapse. For example, moving the tongue forward may address airway closure due to anterior/posterior collapse (i.e., the tongue falls back and closes the airway, and by moving the tongue forward the airway opens). There may be other mechanisms of sleep apnea such as medial/lateral collapse that stimulating the hypoglossal nerve may not be effective at addressing. Some patients may have both anterior/posterior collapse and medial/lateral collapse, resulting in concentric collapse (also called circumferential airway collapse).

Central sleep apnea (CSA) may be yet another mechanism for sleep apnea. CSA may be the result of lack of activation or movement of muscles used for breathing, and may not be due to airway obstruction. Stimulating the hypoglossal nerves may not provide therapy for CSA.

Rather than treating each of the mechanisms for sleep apnea with different medical devices, this disclosure describes example techniques for integrated sleep apnea stimulation from a single IMD to treat the various mechanisms of sleep apnea. As an example, a patient may be implanted with a plurality of leads, where different subsets of the leads may be implanted to stimulate different locations within the patient. Each of the leads may be coupled to the same IMD. For example, a subset of leads may be implanted in the tongue to stimulate the hypoglossal nerve that innervates the genioglossal muscle. Another subset of leads may be implanted to stimulate the ansa cervicalis, which is a loop of nerves, that innervate the infrahypoid muscles in the neck. Another subset of leads may be implanted to stimulate glossopharyngeal nerve, which is the ninth cranial nerve, and sometimes referred to as the CN (cranial nerve) IX. Stimulation of the ansa cervicalis and the glossopharyngeal nerve may provide caudal traction and intrinsic pharyngeal wall tone. Yet another subset of leads may be implanted to stimulate the phrenic nerve to address CSA. For example, implanted to stimulate may refer to the leads being proximate to the nerve such that stimulation signal from the leads stimulates the nerves.

Additional examples of stimulation targets include tensor veli (also called tensor veli palatini), levator veli (also called levator veli palatini), and digastric anterior. For instance, stimulation of the tensor veli tenses the soft palate, and stimulation of the levator veli may elevate the soft palate. Stimulation of the digastric anterior may move the hyoid bone in the anterior direction.

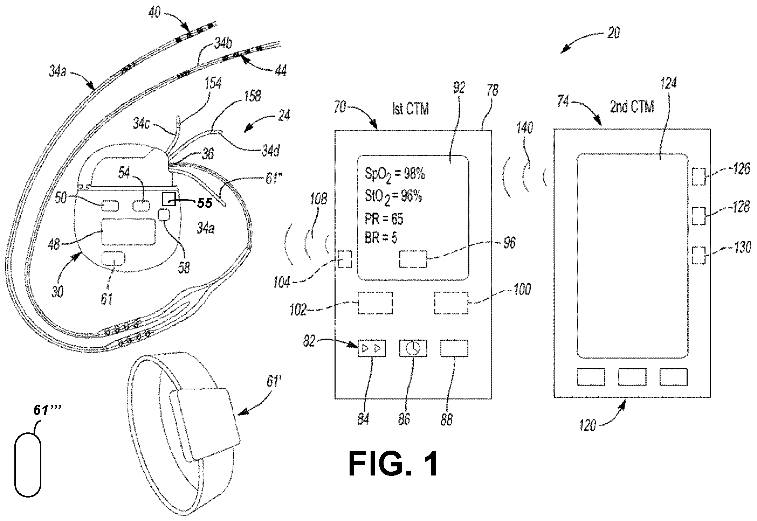

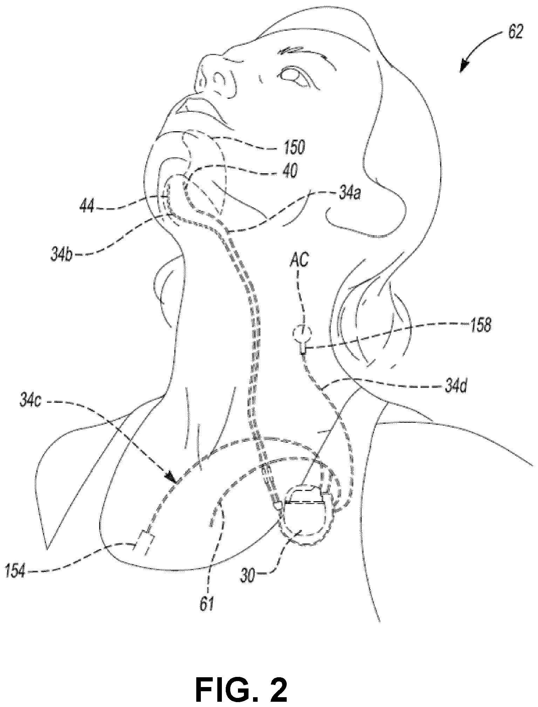

With reference to FIG. 1 and FIG. 2, a stimulation assembly or system 20 is illustrated. The stimulation system 20 may include various components, or selected components of the system 20. In various examples, the stimulation system 20 may be provided for treatment of Sleep Apnea. Sleep apnea may include Obstructive Sleep Apnea (OSA) and/or upper airway restrictive/resistance syndrome (UARS) and/or Central Sleep Apnea (CSA). The stimulation system 20 may include various features and/or systems for the treatment, such as an implantable stimulation assembly 24 that may include, according to various examples, an implantable device (ID) 30 (e.g., an implantable medical device (IMD)). The ID 30 may be implanted and operated (e.g., by instructions stored therein) to provide stimulation to selected muscles and/or nerves for treatment of different mechanisms of sleep apena, such as OSA, CSA, and/or a combination thereof. As discussed herein, a combination of OSA and CSA may also be referred to as multiple or comprehensive Sleep Apnea (MSA). Stimulation of a lingual muscle (i.e. tongue) of a subject 62 may assist in treatment of OSA, while stimulation of a Phrenic nerve (PN) or the diaphragm may assist in treating CSA. The stimulation may be provided to ensure maintaining an open airway and/or re-open an airway and/or cause movement of the diaphragm.

However, stimulation of lingual muscles or stimulation the phrenic nerve may be insufficient to treat sleep apnea, especially if subject 62 experiences sleep apnea from various causes. For instance, stimulating the lingual muscle to move the tongue forward may treat anterior/posterior collapse of the airway. However, stimulating the lingual muscle to move the tongue forward may not treat medial/lateral collapse.

In some examples, ID 30 may be configured to deliver stimulation to an ansa cervicalis, a glossopharyngeal nerve, a tensor veli, levator veli, and/or digastric anterior of the patient, in addition to stimulation to the lingual muscle and/or phrenic nerve to treat the various ways in which subject 62 may experiences sleep apnea.

The ID 30 may be implanted in the subject 62, as discussed herein. Interconnected with the ID 30, as a part of the implantable stimulation assembly 24, may be one or more lead assemblies 34. Various different one or more of the leads 34 may be directed to or implanted in different, spaced apart, or distinct areas of the subject 62. Each of the various leads may be identified with a lower case letter. In various embodiments, for example, a lead assembly 34a,b may be connected to the ID 30 at a selected one of more of a connection point or port 36 and extend to a first lead or stimulator portion (also referred to as a lead end that may include one or more electrodes) 40, and a second lead or stimulator portion (also referred to as a lead end that may include one or more electrodes) 44. In various examples, stimulation may be provided to the subject through the lead ends 40, 44 to treat the OSA of the subject 62. The two lead ends 40, 44 may be used in selected or varying manners to reduce or eliminate fatigue of the lingual muscle to assist in increasing efficacy or success of a treatment.

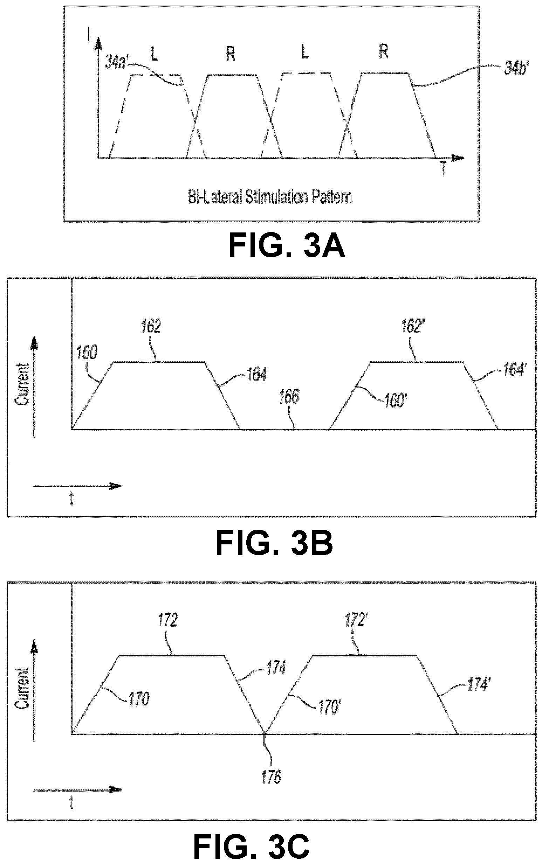

Stimulation at or near the hypoglossal nerve may have a selected therapy or stimulation pattern. As illustrated in FIG. 3A, the stimulation may be bilateral, in other words stimulation may be provided to both sides of a tongue 150 in a selected manner. FIG. 3A is an example of bilateral stimulation using overlapping patterns with a 60% on and 40% off duty cycle, with a ramp up and a ramp down for each. Both sides of the tongue 150 (illustrated in FIG. 2), such as the right left side 34a' and the right side 34b' may have the same, but offset duty cycle. This duty cycle may provide continuous muscle tone to keep the airway open and at the same time avoid muscle fatigue.

The lead or lead assemblies 34 may further include leads that may be connected to the ID 30 and positioned or having stimulation portions at various positions within the subject 62 other than in the lingual muscles, such as near the tongue 150. In various examples, for example, a lead 34c may extend to a portion of the subject 62, such as at or near a diaphragm and/or near a phrenic nerve (PH), within the subject 62. The phrenic nerve (PH) may stimulate or direct a stimulation signal to the diaphragm of the subject 62. Accordingly, stimulating the phrenic nerve may cause movement or contraction of the diaphragm. Contraction of the diaphragm may be lacking during CSA. Therefore, the lead 34c may be positioned to stimulate the phrenic nerve (PN) of the subject 62.

The phrenic nerve PN may be stimulated with the lead 34c. With reference to the FIGS. 3B and 3C, the phrenic nerve may be stimulated according to a selected pattern and/or according to a plurality of patterns. For example, as illustrated in FIG. 3B, the phrenic nerve may be stimulated with a fast contraction (i.e., short ramp up time period to a peak) with an OFF period. The stimulation of the phrenic nerve may cause a contraction of the diaphragm. Accordingly, an ON time and an OFF time may mimic a breathing pattern. As illustrated in FIG. 3B, the stimulation may include a ramp up period 160, a peak period 162, and a ramp down or falling period 164. The stimulation pattern may then include an OFF period 166 before a sequential or next ramp up 160', peak 162', and falling 164'. The graph of FIG. 3B illustrates an amount of current being applied over time. The stimulation to the phrenic nerve that causes a contraction of the diaphragm may occur over a selected period and in a pattern as illustrated in FIG. 3B. In various examples, a modulation may be altered to achieve a selected therapy. Nevertheless, the peak hold time 162 may generally be about 1 to about 3 seconds, including about 2.5 seconds, with a ramp-up /down 160/164 each of about 0.8 second. An OFF time may be about 1 second to about 3 seconds, including about 2 seconds. In various examples, the stimulation pattern illustrated in FIG. 3B may provide for about 10 breaths per minute.

The phrenic nerve may also be stimulated in an alternative or additional pattern, such as that illustrated in FIG. 3C. In FIG. 3C a rise period 170 may be followed by a peak period 172 and a ramp down or falling period 174. The falling period 174 may be followed by an immediate rising period 170' where no gap or substantially no gap 176 is provided between the end of the fall 174 and the rise 170'. The rise 170' may be followed by a peak 172' and further ramp down or falling period 174'. Therefore, the pattern illustrated in FIG. 3C may include little or no gap between one fall period and a subsequent rise period to mimic an immediate inhalation after an exhalation. As illustrated in FIG. 3C, the hold time may be substantially similar to the hold time of the stimulation pattern illustrated in FIG. 3B, but may have a longer rise and fall time such as about 1 second rather than about 0.8 seconds for the rise and fall time of the stimulation pattern illustrated in FIG. 3B.

Accordingly, as discussed above, the rise and fall times may be altered to a selected peak stimulation. The peak stimulation may also be altered relative to a user and/or a selected therapy. In various examples, the peak current may be 8.1 milliamperes (mA). Further, the stimulation may be provided in an appropriate manner, such as to only the right phrenic nerve, only the left phrenic nerve, or bilaterally. As described above, the stimulation to the hypoglossal nerve may be bilateral and, therefore, the stimulation to the phrenic nerves for the diaphragm may also be bilateral. The stimulation, however, may be substantially simultaneous and the same for both phrenic nerves and/or may have an alternating duty cycle similar to that illustrated for the hypoglossal nerve in FIG. 3A. Nevertheless, the phrenic nerve may be stimulated to cause activation of the diaphragm.

In addition, the stimulation system 24 may include a stimulation lead 34d. The stimulation lead 34d may be positioned to stimulate selected muscles or portions of the upper airway, such as strap muscles or other muscles in the upper airway. The lead 34d may be provided to stimulate selected nerve groups or bundles, such as an ansa cervicalis (AC). The AC may cause contraction or stiffening of selected muscles within the upper airway. The muscles stimulate or contracted by the AC may include those that limit or reduce medial to lateral collapse within the upper airway. The AC stimulation, therefore, may cause or reduce collapse that may lead to OSA. Stimulation of certain roots and/or portions of the AC may activate certain muscles as disclosed in Kent DT, Zealear D, Schwartz AR, Ansa Cervicalis Stimulation: A New Direction in Neurostimulation for Obstructive Sleep Apnea, CHEST (2020), doi: https://doi.org/10.1016/j.chest.2020.10.010, WIPO publication WO WO2020185549 and/or U.S. Pat. App. Pub. No. 2020/0069947.

The stimulation of the AC with the lead 34d may be performed in an appropriate manner. As discussed above, the stimulation to the leads 34a, b and/or the lead 34c may be according to a selected pattern. The AC may be stimulated in a similar manner and may include a selected duty cycle, such as similar to those discussed above. Thus, the stimulation may include an appropriate ramp up, peak, and ramp down time to achieve an appropriate stimulation and contraction of the muscles stimulated or innervated with the AC. The AC may be stimulated according to a selected stimulation pattern that may be recalled from a memory, as discussed above, and/or selected and provided to the memory by the user. The stimulation pattern for the AC may be similar to those illustrated in FIGS. 3A-3C.

In one or more examples, stimulation parameters (e.g., wave shape, frequency, pulse width, and amplitude) for stimulating the hypoglossal nerve(s), the phrenic nerve, the AC, and other example stimulation locations described in this disclosure may be similar. However, in some examples, the timing of the stimulation to different locations may be different. For example, it may be possible to synchronize the stimulation of the hypoglossal nerves with the stimulation of the other locations. As another example, it may be possible to synchronize opposite sides of the airway in different muscles. For instance, if there are additional leads implanted to stimulation different muscles of tongue 150 (FIG. 2), it may be possible to stimulate left hypoglossal nerve and right lateral muscle together, and stimulation right hypoglossal nerve and left lateral muscle together. There may be overlap in the stimulation as well (e.g., ¼ cycle offset).

In various examples, therefore, the lead 34 which may include and/or be referred to as the leads 34a-34d, may be individually and/or collectively connected to the ID 30. In various examples, the leads 34a, 34b may be used to stiffen or reduce the collapse of muscles or muscle groups that may cause anterior to posterior collapse. The lead 34d may stimulate muscles that reduce medial to lateral collapse. Therefore, the leads 34a, 34b, 34d may reduce or eliminate collapse of the upper airway. The lead 34c may be used to assist in ensuring activation of the diaphragm, such as by stimulating the PN, to treat CSA. Therefore, the same, single ID 30 may include leads that are directed or connected to a plurality of sites or locations within the subject 62 to treat a plurality of distinct or separate conditions related to sleep apnea.

Stimulation patterns for each of the leads may be construed to coordinate the stimulation together to optimize the therapy for the subject, as discussed herein and above. One example may be to stimulate at or near the hypoglossal nerve (HGN) at the same time as another therapy, such as stimulating the AC. Another example could be to temporarily stimulate at or near the HGN and AC stimulation to a high level if the upper airway has collapsed, at the same time stopping the phrenic nerve stimulation to facilitate reopening of the airway. Once the airway has reopened at or near the HGN and AC stimulation return to normal levels and the phrenic nerve stimulation resumes.

The ID 30 may also include various components such as a power source (e.g., a battery) 48. The battery 48 may be rechargeable and recharged in a selected manner, such as through inductive recharging. The battery 48 may be charged via a wired or wireless charging system. Wireless or contactless recharging modalities may include inductive recharging, resonant recharging, etc. Exemplary stimulation systems that include rechargeable batteries include the Intellis^{®} implantable neuro stimulator sold by Medtronic, Inc. having a place of business in Minneapolis, Minnesota. It is understood, however, that any appropriate power storage system may be provided, and the battery is merely exemplary. The power source 48 is to provide a selected current and/or voltage between one or more electrodes of the lead assembly 34.

The ID 30 may further include selected components to assist in providing stimulation through the lead assembly 34. For example, the ID 30 may include a processor 50 that may be any appropriate processor to execute instructions provided to the ID 30 from an external source and/or saved on a memory 54. The memory 54 may be any appropriate memory system. Further, the ID 30 may include a selected transceiver system or transceiver 58 that may be used to transmit and/or receive information and/or instructions from an external source. The transceiver 58 may be any appropriate transceiver such as those discussed further in. As also illustrated, ID 30 may include stimulation circuitry 55 configured to transmit stimulation signals to lead 34.

The stimulation assembly 20 may include, according to various examples, the implantable stimulation portion 24 as a stand-alone system. In various examples, the memory 54 may include any appropriate instruction and/or algorithms for operation of the stimulation portion 24. The processor 50 may execute the instructions from the memory 54 and the battery 48 may be provided to power the stimulation portion 24. It is understood, however, that various portions may also be provided external to a subject 62 (FIG. 2) to assist in interacting with the ID 30.

Various sensors may be provided to transmit and/or receive a signal regarding the subject 62. A sensor 61 may be included with the ID 30 (e.g., formed on an exterior of the ID 30, placed within the ID 30, and/or connected to the ID 30). The sensor 61 may include one or more of an accelerometer, a global positioning system (GPS), oximeter, electromyography sensor, pressure sensor, impedance sensor, etc. The sensor 61 may be used to assist in determining selection of treatment for the subject 62. It is understood that the sensor 61 may also communicate with other systems. For example, a combination of measurements/sensing of accelerometry with impedance enables a spectroscopy detection algorithm to differentiate between OSA, CSA, mixed apnea (i.e., both anterior/posterior collapse and CSA), or no apnea. Concentric collapse (i.e., inclusive of both anterior/posterior and medial/lateral) may have a detectable unique signature to facilitate feedback loop to appropriately turn ON/turn OFF therapy (e.g., stimulation) to all of leads 34a, 34, and 34d.

Alternatively or in addition to the sensor 61, an external or wearable sensor 61' may be placed on the subject 62. The wearable sensor 61' may be any one or more of the same sensors as discussed above, including redundant thereto. The wearable sensor 61' may also include additional or different sensors. Sensors, particularly external sensors, may include a position (e.g. relative to gravity) sensor, temperature sensor, CO₂ detector, airflow (e.g. nasal or mouth) detector, microphone (e.g. for detecting breathing sounds (like snoring), impedance detector (e.g. to detect lung volume). Further, various sensors may be used to determine or monitor "quality of sleep" such as sensing via EEG or integration of various sensors already mentioned. A quality of sleep determination may be correlated to OSA therapy effectiveness. The external sensor may be fixed to the subject in any appropriate manner, such as a write strap, a chest strap, and/or an adhesive portion (e.g., tape). Regardless of the type, the sensor in the wearable sensor 61' may also communicate with the ID 30 and/or other portions of the system 20.

A sensor 61" may also be implanted in the subject 62 remote from the ID 30. For example, an accelerometer may be positioned near a sternum of the subject 62. The accelerometers may be in communication with the ID 30, such as with a wire or lead. The sensor 61", therefore, may be implanted and/or fixed with the subject 62 but at a location remote for the ID 30.

Another example of a sensor is sensor 61'''. Sensor 61'" may be microsensor that is implanted within subject 62 and is configured to sense temperature, oxygen (e.g., is an oximeter), accelerometer, GPS, electromyography sensor, pressure sensor, impedance sensor, etc. For instance, sensor 61'" may be similar to sensor 61 or sensor 61" but may be external to ID 30 and not in communication with ID 30 through a wire or lead. In one or more examples, sensor 61''' and ID 30 may communicate wirelessly. Sensor 61'" may be implanted near the lungs, in the neck, or various other locations. The small form factor of sensor 61'" may be small enough to allow implantation in locations throughout subject 62.

In various examples, external systems, as a part of the stimulation assembly 20, may include a first external controller or transceiver 70, referred to as communication telemetry module (CTM) 70 and may communicate with ID 30. CTM 70 may be referred to as first control module 70. In various examples, the CTM 70 may also be a recharge system and may include a monitor that will predict battery usage and recharge times based on stimulation usage of the subject 62. Low battery conditions will warn the patient (e.g., subject 62) and allow the patient to select "stimulation priorities" of selected leads to conserve battery power.

The stimulation assembly 20 may further include a second transmission and/or control module (CTM) 74. The second control module 74 may communicate with the first control module 70, may communicate directly with the ID 30, and/or may communicate with the ID 30 through the first control module 70. In some, but not all, examples, only one of the first control modules 70 or the second control module 74 may be provided for the stimulation assembly 20.

The first CTM 70 may be provided to the user 62 for a personal and/or at home use. The CTM 70 may be used by the user 62 to assist in controlling the ID 30 to provide stimulation to the user 62. The CTM 70 may also be used to alter operation of the ID 30, such as by the user 62, input indications from a user 62, or other appropriate mechanisms.

The CTM 70 may include a body or module portion 78 that is sized to fit within a hand of the user 62. Accordingly, the CTM 70 may be mobile relative to the user 62 and/or allow for ease of transport and use by the user 62. Further, the CTM 70 may be positioned near the user 62 at any appropriate time, such as during a selected or near a sleep period of the user 62. The CTM 70 may further include one or more input portions, such as a physical button (also referred to as a hard button) or hard selection assembly 82 that may include one or more buttons that allow the user 62 to input operation or select operations of the ID 30. For example, the hard buttons 82 may include a start and stop button 84, a timer button 86, and/or a power or emergency button 88. The CTM 70 may also include a selected display 92 to provide information to the user 62 such as a pulse rate, oxygen saturation value, breathing rate, sleep time and/or reported/determined quality, or other appropriate information. The display 92 may provide information, such as historical information, to a clinician for selecting programming and operation of the ID 30. The information may include raw and/or analyzed data of the subject 62 and/or a population of subjects. The display 92 may also be a touchscreen or touch sensitive and, therefore, include one or more soft buttons.

The CTM 70 may include various components, such as a processor 100, a memory 102, and a transceiver 104. As illustrated in FIG. 1, the CTM 70 may transmit a signal, such as a wireless signal 108 to the ID 30. Further, the ID 30 may transmit a signal, such as a wireless signal 110, to the CTM 70. Accordingly, the CTM 70 may receive information from the ID 30 and/or transmit information to the ID 30, and vice versa. The CTM 70 may be used to program or select operation of the ID 30 and the ID 30 may include sensors to transmit information to the CTM 70. The CTM 70 may be configured as any appropriate device and/or may be incorporated as an application with an appropriate mobile computer (e.g., mobile phone, tablet, etc.). The application may be made for any appropriate operating system such as the iOS operating system, Android^{®} operating system, etc. Also, the CTM 70 may be powered in an appropriate manner, such as with an internal battery that may be charged via a wired or wireless charging system. Wireless or contactless recharging modalities may include inductive recharging, resonant recharging, etc.

The stimulation assembly 20 may further include a second control module, such as a CTM 74. The CTM 74 may include components similar to the first CTM 70. The CTM 74, however, may be a larger and/or permanent device provided with a clinician for operation of the ID 30 and/or programming of the first CTM 70. The second CTM 74 may generally be non-mobile, in other words not intended to be moved with or by the subject 62. Accordingly, the second CTM 74 may include various hard buttons 120, a touch screen 124, a processor 126, a memory 128, and a transceiver 130. Thus, the CTM 74 may transmit a signal, such as a wireless signal 140, to the first CTM 70 and/or the ID 30. Thus, the ID 30 may also transmit the signal 110 to the CTM 74 and/or the first CTM 70 may transmit a signal to the second CTM 74. The CTM 74, in various examples, may include a distributed or "cloud" system and/or processing. The processing may include processing separate from any local subject system.

Accordingly, the simulation assembly 20 may include various components for operation of the ID 30 and in various applications. The ID 30 may be implanted in the subject 62 for stimulation of the subject 62 according to a selected mechanism or method. Various control components may be provided at selected times to allow for programming of the ID 30, altering of the programming of the ID 30, receiving output (e.g., historical sensor data) from the ID 30, and analysis of operation of the ID 30, historical data, and other information. In this manner, the first CTM 70 may be provided for substantially immediate use and/or adjacent use during operation and/or after implantation of the ID 30. The second CTM 74 may be provided for initial programming, follow up, and interaction with the subject and/or a central data storage and/or analysis system by selected users, such as the clinician.

With continuing reference to FIG. 1 and additional reference to FIG. 2, the stimulation portion 24 may be implanted into a selected subject 62. As illustrated in FIG. 2, the selected subject 62 may be a human subject. It is understood, however, that any appropriate subject may have the stimulation portion 24 positioned therewith at appropriate times. It is further understood that the ID 30 may be positioned near or external to the subject 62 such that the lead portion 34 is implanted percutaneously into the subject 62 and the stimulation portion 30 (e.g., power and control) is exterior to the subject. Therefore, the discussion herein of an implantable device 30 will not be understood to eliminate selected components, such as a power source, stimulation reconnection, or other features or portions that may be placed in an external device for percutaneous transmission through the lead assembly 34 to a stimulation location of the subject 62.

With continuing reference to FIG. 2, the ID 30 may be implanted in the subject 32 in any appropriate location, such as in an abdominal wall, a chest wall, sub-dermally near a clavicle, or other appropriate locations. The lead assembly 34 may be connected to the ID 30 and pass through selected tissue to a selected location for stimulation of the subject 62. In various examples, as illustrated in FIG. 2, the stimulation lead assembly 34a, b, including the first and second lead tip portions 40, 44 may be positioned in a lingual tissue (i.e., a tongue 150). The lead assembly 34 may be positioned in the subject 62 along a single path for a selected portion of the length of the lead assembly 34a, b and/or may be immediately divided into two lead portions for positioning in the tongue 150. The tongue 150 is formed of a plurality of muscle portions that may act in concert to cause movement of the tongue 150. Accordingly, reference to the tongue 150 is understood to refer to relevant portions of the tongue 150. In addition, one skilled in the art will understand, the tongue 150 may include a plurality of nerve portions or constructs, including nerve ends, endplates, etc.

Tongue 150 includes one or more branches of two hypoglossal nerves. There are two hypoglossal nerves in subject 62, and each of the hypoglossal nerves terminates at a motor point junction or endplate near the tip of tongue 150. The lead contacts 40, 44 may be placed in contact with or near the hypoglossal nerves. In some examples, rather than having cuff electrodes that cuff around the hypoglossal nerves at a more distal location of the hypoglossal nerves, relative to tongue 150, lead contacts 40, 44 may be ring electrodes that are proximate to the hypoglossal nerves.

The stimulation to the hypoglossal nerves may cause stiffening and/or extension of the tongue. The stiffening or stimulation of the tongue may eliminate or reduce anterior to posterior collapse of the tongue 150 and related muscles.

Additionally, the lead assembly 34 may include the lead portion or lead assembly 34c. The lead 34c may also be connected to the ID 30 and have lead portions or contacts 154 positioned at or near the phrenic nerve PN. As illustrated in FIG. 2, the phrenic nerve PN may extend through an upper torso of the subject 62. In various conditions, central sleep apnea (CSA) may be caused due to a lack of innervation or activation of the diaphragm muscle. During CSA, the phrenic nerve may not be activated or stimulated properly for various reasons. For example, a nerve signal may not be generated at or within the brain that is transmitted through or to the phrenic nerve. Further, a blockage or other damage may have occurred to the phrenic nerve. Additionally various issues may arise regarding the diaphragm where a greater or selected type of stimulation is needed or selected to achieve a selected movement of the diaphragm.

The lead 34c, therefore, may extend from the ID 30 to at or near the phrenic nerve PN. The contact 154 may be placed near and/or in contact with the phrenic nerve PN to provide stimulation to the phrenic nerve PN. The stimulation to the phrenic nerve PN may cause the diaphragm to activate and the subject 62 to inhale or exhale. By contracting or activating the diaphragm muscle the subject 62 may be caused to breathe. Regardless, stimulation of the phrenic nerve PN may assist in causing activation of the diaphragm to assist in breathing and treat CSA.

The lead assembly 34 may further include the lead 34d, as discussed above. The lead 34d may extend from the ID 30 to be positioned near the ansa cervicalis (AC), as illustrated in FIG. 2. The lead 34d may also include a lead contact or contacts 158. The contacts 158 may be positioned at or near a selected portion of the AC for stimulating or stiffening selected portions of muscle groups within the upper airway. In various examples, the contacts 158 may be included in a cuff electrode. In various examples, the leads 158 may also be provided and/or alternatively be provided as ring or pad electrodes that are positioned on or near the AC. The electrodes of the contact 158, for example, may be positioned on the exterior of a lead portion for positioning near or in contact with the AC. Nevertheless, simulation may be provided through the lead 34d to stimulate the AC.

The stimulation of the selected portions of the subject is 62 may be to assist in treating or eliminating various mechanisms of sleep apnea. The stimulation of various muscles may reduce or eliminate collapse into an upper airway, such as by stimulating muscles in the tongue 150. Selected muscles may be stimulated at or near the hypoglossal nerve. Stimulation may include that disclosed in U.S. Pat. App. Pub. No. 2021/0228871 A1 (U.S. patent application 16/752,236 filed on January 24, 2020). Stimulation of the tongue 150 and/or associated muscles may operate to stiffen or contract these muscles to reduce or eliminate anterior to posterior collapse within the upper airway. The obstruction caused by the collapse may cause obstructive sleep apnea (OSA). The obstructive sleep apnea caused by this muscle group collapse may be treated by stimulating these muscles or nerves relative to these muscles to achieve movement of these muscles out of the upper airway.

The ansa cervicalis (AC) may also be stimulated with the contacts 158 from the lead 34d. These contacts 158 may provide stimulation to the AC to assist in contracting or stiffening muscles (e.g., strap muscles) within the upper airway. The muscles innervated by ansa cervicalis are generally the sternohyoid, sternothyroid, and omohyoid muscles. The stimulation of the AC with the selected lead 34d is the activation of these muscles to cause Caudal traction (i.e., stretching the airway) (ansa cervicalis) and intrinsic pharyngeal wall tone (glossopharyngeal nerve) mechanisms for upper airway support.

These muscles may collapse medial to lateral within the upper airway and also cause OSA. Accordingly, OSA may occur due to collapse of various muscles into the upper airway including muscles that collapse anterior to posterior and/or those that collapse medial to lateral. Regardless the collapse of the muscles and the upper airway may cause the OSA.

Accordingly, stimulation of selected muscle groups may eliminate or reduce the collapse into the upper airway. As noted above, activating muscles in or near the tongue and/or strap muscles may assist in reducing or treating the collapse into the upper airway that causes OSA. In other words, OSA may be reduced or treated within the subject 62 by stimulating the muscle groups or the nerves to the muscle groups.

The above describes some example techniques to stimulate muscle groups or nerves to the muscle groups to address OSA. However, there may be additional areas where ID 30 may stimulate subject 62. As a few examples, ID 30 may be configured to stimulate at least one of a glossopharyngeal nerve, tensor veli, levator veli, and/or digastric anterior of subject 62 (e.g., patient 62). For instance, similar to how leads 34 are implanted in subject 62, leads that extend to the glossopharyngeal nerve, tensor veli, levator veli, and/or digastric anterior may be implanted and coupled to ID 30 to stimulate respective locations within subject 62. Leads that extend to the glossopharyngeal nerve, tensor veli, levator veli, and/or digastric anterior may be implanted using a probe, needles, and canula similar to leads 34 for stimulating other locations of subject 62.

Accordingly, in one or more examples, ID 30 may be coupled to at least a first lead and a second lead. For example, stimulation circuitry 55 may be coupled to the first lead and the second lead. Stimulation circuitry 55 may include current sources and sinks coupled to electrodes of leads 34 for independently controlling the current delivered to the various nerves. In some examples, stimulation circuitry 55 may include voltage sources to deliver voltage and control the amount of stimulation delivered to the various nerves.

Processor 50 may cause stimulation circuitry 55 of ID 30 to transmit a first stimulation signal to the first lead to stimulate at least one of an ansa cervicalis, a glossopharyngeal nerve, tensor veli, levator veli, and digastric anterior of a patient, and transmit a second stimulation signal to the second lead to stimulate at least one a hypoglossal nerve or a phrenic nerve of the patient. In this way, the same ID 30 may be configured to treat different mechanisms of sleep apnea, providing a more complete sleep apnea treatment while minimizing surgical time, and as described below, to provide quick, dynamic changes to treatment as needed.

For instance, the first stimulation signal delivered by stimulation circuitry 55 may be configured to cause stimulation, contraction, and/or traction of at least one of a sternhyoid, a sternothyroid, or an omohyoid muscle of subject 62 (e.g., by stimulating the ansa cervicalis and/or glossopharyngeal nerve). As another example, the first stimulation signal delivered by stimulation circuitry 55 may be configured to cause a soft palate of subject 62 to tense and/or elevate (e.g., by stimulating the tensor veli and/or levator veli).

As described above, the second stimulation signal delivered by stimulation circuitry 55 to the second lead may be to stimulate at least one a hypoglossal nerve (e.g., to prevent anterior/posterior collapse) or a phrenic nerve (e.g., to prevent CSA) and cause the diaphragm to move. In some examples, processor 50 may be configured to cause stimulation circuitry 55 of ID 30 to stimulate both the hypoglossal nerve and the phrenic nerve. As an example, the second stimulation signal delivered by stimulation circuitry 55 may be configured to stimulate one of the hypoglossal nerve or the phrenic nerve. Stimulation circuitry 55 of ID 30 may be coupled to a third lead, and processor 50 may be configured to cause stimulation circuitry 55 of ID 30 to transmit a third stimulation signal to the third lead to stimulate the other of the hypoglossal nerve or the phrenic nerve.

ID 30 may output the various stimulation signals in an alternating way or simultaneously. For example, processor 50 may cause stimulation circuitry 55 of ID 30 to alternate the first stimulation signal (e.g., to an ansa cervicalis, a glossopharyngeal nerve, tensor veli, levator veli, and/or digastric anterior) and the second stimulation signal (e.g., to a hypoglossal nerve or a phrenic nerve). For instance, stimulation circuitry 55 of ID 30 may first transmit the first stimulation signal, and after the first stimulation signal is complete, stimulation circuitry 55 of ID 30 may transmit the second stimulation signal.

As another example, processor 50 may cause stimulation circuitry 55 of ID 30 to simultaneously transmit the first stimulation signal and the second stimulation signal. In some examples, the amplitude, pulse width, and frequency ranges of the first stimulation signal and the second stimulation signal may be the same. However, the timing of the first stimulation signal and the second stimulation signal may be different, but may be related to one another. For example, the timing of the first stimulation signal may define the timing of the second stimulation such that the subject 62 experiences effective treatment for concentric collapse. Such effective treatment for concentric collapse may occur with the first and second stimulation signals overlapping for some subjects, but may occur with the first and second stimulation signals at different times for some subjects.

For example, stimulation circuitry 55 of ID 30 may transmit the first stimulation signal and the second stimulation signal at the same time. As another example, stimulation circuitry 55 of ID 30 may transmit the first stimulation signal, and before the first stimulation signal is complete, stimulation circuitry 55 of ID 30 may transmit the second stimulation signal (e.g., there may be some overlap in the first stimulation signal and the second stimulation signal).

The stimulation system 20, therefore, may be provided relative to the subject 62 to cause stimulation of one or more portions of the subject 62. For example, as discussed above, the sensors 61, 61', 61" and/or 61'" may sense various parameters regarding selected or different conditions of the subject 62. The conditions of the subject may include those that are generally monitored or sensed during polysomnography (PSY). During PSY a user, such as a sleep study agent, may study the subject 62. The PSY may be used to read or determine various conditions of the subject 62 during a selected sleep cycle. The PSY may be used to assist in determining a treatment of the subject 62. Accordingly, the sensors 61, 61', 61", and 61''' may sense all or parts of the features studied during a PSY.

It is further understood that various sensors may be provided at the different locations with the lead or lead contacts such as accelerometers, pressure sensors, and the like. When present, the sensors positioned at or near the ends of the leads 34 may assist in determining status of various parameters of the subject 62, such as muscle stiffness or tone and other parameters. Nevertheless, the sensors 61 may sense parameters of one or more conditions of the subject 62. For example, an accelerometer may be used to determine the amount of breathing or rate or type of breathing of the subject 62. Other sensors may also be used to attempt to determine the breathing status, alertness, or the like of the subject 62.

Having a single ID 30 that is configured to treat various mechanisms of sleep apnea may be beneficial as compared to having multiple different devices that each treat different mechanisms of sleep apnea. As described above, one benefit of having a single ID 30 may be less surgical time. Another benefit of having a single ID 30 may be feedback control of determining stimulation parameters for one location based on sensing in other locations. For example, if sensors 61 indicate that subject 62 is having a hard time breathing, then ID 30 can automatically adjust stimulation parameters in different locations (e.g., stimulation delivered by contacts of lead 34d and contacts of leads 34a, 34b). As another example, if sensors 61 indicate low tone in a neck muscle, indicative of medial/lateral collapse, in addition to controlling stimulation parameters at the ansa cervicalis, to address the medial/lateral collapse, ID 30 may automatically start stimulation or automatically increase stimulation to the hypoglossal nerve to ensure that there is no anterior/posterior collapse. By having a single ID 30 configured to provide stimulation to different locations and address different mechanisms of sleep apnea, there may be a higher likelihood of dynamically and quickly providing stimulation to different locations to minimize the impact of the apnea.

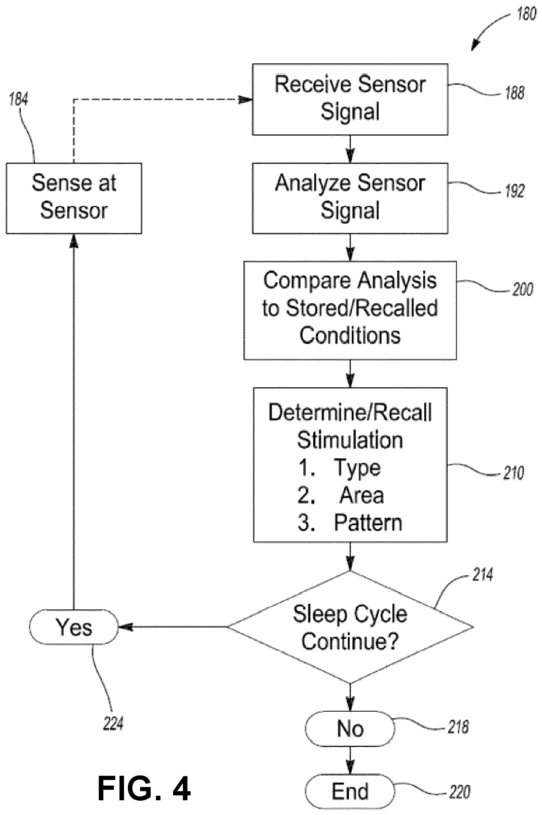

With continuing reference to FIGS. 1 and 2, and additional reference to FIG. 4, a process 180 of operation of the stimulation system 20 is illustrated. As discussed above, the sensors 61 (understood to include one or more of the sensors 61, 61', 61", and 61''') may be positioned to sense various parameters of the subject 62. The sensors 61 may generate a signal in block 184 that is sent to the processor 50 of the ID 30 and/or transmitted to one of CTM's 70, 74. Regardless a sensor signal may be received in block 188. After receiving the sensor signal in block 188, an analysis of the sensor signal may occur in block 192.

The analysis of the sensor signal in block 192 may occur with the processor 50 of the ID 30. In various examples, the ID 30 may be substantially self-contained and be able to receive sensor input, analyze sensor input, and provide selected simulation. It is understood, however, the discussion of the processor 50 may also refer to analysis or execution of instructions by processors external to the ID 30.

Analysis of the sensor signal block 192 may include analyzing sensor signals or inputs outside of a selected threshold, initiation of selected or predetermined factors (e.g., a physical position of the subject 62). In various examples, the sensors 61 may include a pulse oximeter. The pulse oximeter may send the signal regarding the measured or sensed blood oxygen level of the subject 62. Accordingly, analysis of the sensor signal in block 192 may include a determination of the blood oxygen level and its comparison or value within a selected range and/or threshold.

As one example, for a pulse oximeter a drop of 10% in O2 blood saturation could trigger a change in therapy stimulation. This change could include an activation or increase in amplitude of stimulation of the hypoglossal nerve(s), phrenic nerve, and other nerves (e.g., AC) to activate muscles of the upper airway. A change of physical position could be going for laying on the front to laying on the back. The worst position tends to be laying on the back. This could be unique for each patient and would be determined by testing in a sleep study. Other sensors could include sensing snoring, or a change in breathing pattern. Accordingly, the analysis may include a determination of whether the patient is sleeping on back or not, and if there are snoring episodes above a certain threshold or change in breathing pattern.

The analysis of the sensor signal in block 192 may thereafter and/or simultaneously be compared to a stored or recalled of selected parameters in block 200. As discussed above, the ID 30 may include the memory 54 and/or may access memory such as in the CTM 70. This may allow the analysis in block 192 to be compared to recalled or stored values. For example, the blood oxygen level may be compared to a selected threshold or value. Further, the breathing rate or selected pressure signals may also be compared to predetermined or stored values.

The analysis in block 192 and the comparison in block 200, therefore, may be used to determine or recall stimulation factors or parameters in block 210. The recall of stimulation parameters in block 210 may include various parameters, such as those discussed herein. For example, the comparison may include a determination of a type of sleep apnea, such as OSA, CSA, or an inclusive sleep apnea (ISA). The analysis and comparison of the sensor signals may be used to determine the type of SA occurring. Accordingly, the determination and recall stimulation may include determining a type of SA that occurs.

Further, the recalled type of SA may be used to assist in determining a type of stimulation, an area to be stimulated, and/or a pattern of stimulation. Various parameters may be sensed with the sensor 61 and/or analyzed to determine the SA. For example, various signals may be used to determine sympathetic and/or parasympathetic tone (e.g., heart rate variability and blood pressure). Signals may measure depth of sleep such as EEG, signal to measure if a person is snoring (e.g., a microphone), a signal to measure how much movement a patient has (e.g., with an accelerometer). These various parameters may all be used and/or be selectively used to determine the SA.

One or more physiological signals may be sensed with one or more of the sensors 61. For example, one or more sensors 61 may sense an accelerometer signal, a cardiac electrogram signal (ECG), and/or a bioimpedance signal. The processor 50 may detect a primary biomarker indicating an OSA (including either or both of anterior/posterior collapse and/or medial/lateral collapse), CSA, or a combination OSA/CSA event. Respiration may be used as the primary biomarker, and may be discriminated with one or more signals from an accelerometer, an ECG sensor, or a bioimpedance sensor. In various examples, respiration detection may be based on a signal from one or more of a 3-axis accelerometer sensor, an ECG vector may be taken from the lead 34c to the IMD 30 (e.g., the sensor 61 included therewith), etc. to detect an apnea event.

In some examples, the classification of a type of respiration based on a secondary biomarker and/or may include a series of sequential determinations of whether the respiration signal(s) satisfy criteria for different classifications of SA. There may be various ways in which to classify the SA.

As one example, there are two levels for the classification of SA. The first level is a change in breathing pattern. One determination of classifying the SA is based on whether a hypopnea or apneic event is occurring. Whether the hypopnea or apneic event is occurring could be determined by detecting a change in the breathing pattern. For instance, either breathing stopped or a marked reduction in minuet ventilation (the volume of air being breathed in each breath) is identified. For example, hypopnea is when subject 62 takes in shallow breaths for 10 seconds or longer while asleep and the airflow is at least 30% lower than normal. However, breathing of subject 62 does not totally stop since the airway is only partly blocked. With apnea, the airways are fully obstructed so that subject 62 does stop breathing for 10 seconds or more during the night. With either case, subject 62 might wake up many times during sleep to catch your breath without being aware.

The second level of importance is detecting if the hypopnea or apneic event is causing other physiologic changes (like a reduction in O2 saturation or an increase in sympathetic tone (measured in HR increase) or an arousal (measured in changes in EEG pattern). The algorithm to determine how aggressive the therapy is would be determined of the severity of both those levels. For example, if the first level is not happening then SA is not occurring so there is not a need to treat. If the second level is not occurring there is SA, but it is not too important to treat aggressively because it is not hurting the patient.

In some examples, if processor 50 determines that OSA is detected the processor 50 may, as discussed herein, control therapy delivery circuitry to deliver OSA therapy. The determination may further determine the type of OSA, such as one or both of anterior/posterior collapse and/or medial/lateral collapse. Thus, the therapy, such as stimulation to leads 34a, b and/or lead 34d may be made. Further, if OSA is not detected, processor 50 may determine whether CSA is detected. If CSA is detected, processor 50 may control therapy delivery to deliver therapy configured to treat CSA, such as stimulation with lead 34c. If CSA is not detected, processor 50 may determine whether one or more criteria to classify respiration as mixed OSA/CSA are satisfied and, if so, deliver both OSA and CSA therapy, such as with one of more of the leads 34a, 34b, 34c, or 34d in a selected and/or separate manner.

The input from the sensors may be analyzed by the processor 50 to determine a sleep spectrogram according to one or more examples. OSA and CSA have different physiologic origins, which may be better detected through the creation of a "sleep spectrogram" by morphing or combing several different physiological signals together from the sensors 61 by processor 50. In OSA, the upper airway may become partially or completely blocked while the subject 62 sleeps. In CSA, there is a cessation of respiratory drive resulting in a lack of respiratory movements (e.g., a lack of lower brain stem signal). In mixed OSA/CSA there is a parallel blending of the primary biomarkers. The processor 50 may determine (e.g., combine primary respiration sensor signals) to create a "sleep spectrogram," which may provide increased sensitivity and specificity to discriminate between OSA, CSA, or mixed OSA/CSA manifestations and allow automatic and appropriate therapy delivery. Further, during a sleep study of the subject 62 a normal or healthy sleep may be determined and saved as a base line for the system 20. This may also change over time and be determined by an adaptive or machine learning system of the system 20. SA events may be determined by a deviation from this normal sleep. The type of deviation may be used to determine the type of SA and the selected therapy therefor.

In addition or alternatively, a self-adapting or machine learning control system can be used to learn a response of the subject 62 to various stimulation types and/or patterns. The machine learning system may adapt the stimulation levels, location, pattern, etc. over time as the condition of the subject 62 changes. Thus, the type of therapy may not be limited to only a saved and recalled predetermined therapy type.

In general, optimal stimulation patterns may be tailored to individual patients and their upper airway morphology through stimulation algorithms that automatically test various combinations of various muscles, both in relative temporal variations (e.g., how quickly and how long the muscles maintain tone) and variation of stimulation strength (e.g., how much amplitude is needed to cause the muscle to achieve tone) to maximize the reduction in occurrence of apnea-hypopnea index (AHI).

For instance, for subject 62, a clinician may determine therapy parameters for delivery of stimulation to one or more of an ansa cervicalis, a glossopharyngeal nerve, tensor veli, levator veli, digastric anterior, hypoglossal nerve, and phrenic nerve that results in having the lowest AHI. These therapy parameters may be stored in memory 34. When a sleep apnea event is detected, ID 30 may access the therapy parameters to deliver therapy.

As another example, the clinician may determine therapy parameters that minimize AHI for each of the different mechanisms of sleep apnea. For example, for anterior/posterior collapse, in addition to stimulating the hypoglossal nerve to push tongue 150 forward, there may be benefit in providing some, even if minimal, stimulation, to ansa cervicalis to stiffen neck muscles. This way, even if tongue 150 is not pushed all the way forward to fully open the airway, there is space from the lateral and medical side for air to enter and exit. If processor 50 determines that anterior/posterior collapse is occurring, then processor 50 may determine the stimulation to provide to the hypoglossal nerve and ansa cervicalis to treat the sleep apnea.

For example, if the sleep apnea that is occurring is determined to be OSA (e.g., by comparison to predetermined parameters), stimulation may occur along the leads 34a, 34b and/or 34d. In various examples, the type of collapse in the upper airway may be determined and only a stimulation in areas such as within the tongue 150 and/or at the ansa cervicalis AC may occur. It may further be determined that both areas may be stimulated to reduce or eliminate a circumferential or total collapse of the upper airway. Accordingly, the comparison in block 200 may be used to determine that a stimulation of all or a part of the upper airway leads need to be activated to assist in treating the OSA. It is further understood that the sensor inputs may be used to determine that only one or the other of the muscle groups or areas need to be stimulated with the selected leads.

If the sleep apnea that is occurring is determined to be CSA (e.g., by comparison to predetermined parameters), stimulation may occur along the lead 34c. That is, the inputs may be determined to stimulate the phrenic nerve PN to activate the diaphragm. The ID 30, therefore, may be operated to additional or alternatively only to stimulate with the lead 34c.

In addition, the determined stimulation parameters in block 210 may be to stimulate all areas including stimulation of all of the areas, such as the tongue 150, the AC, and/or the phrenic nerve PN separately and/or together. Each of the stimulation areas or regions may be separate, distinct, and/or spaced apart. The stimulation of all the areas may be substantially simultaneous, sequential, or at a selected pattern to achieve a selected breathing rate and type of the subject 62. For example, the lead 34c may be used to stimulate the phrenic nerve PN followed by stimulation of the ansa cervicalis AC and again followed by a simulation within the tongue 150 by the leads 34a, 34b. It is understood, as discussed above, an appropriate pattern may be provided to stimulate the subject 62, however, with the ID 30 through one or more of the various lead assemblies 34.

The stimulation of the various portions of the subject 62 may also be according to a selected pattern, including the pattern as discussed above. Further the pattern may include a type of stimulation, such as a selected wave form of stimulation of the subject 62. The waveform for stimulation may be included in selecting a type of pattern, such as areas of the subject to simulate in what order, in combination.

The ID 30 thus may be used to treat multiple types of SA, also referred to as inclusive sleep apnea. The inclusive sleep apnea may include different and non-localized sleep apnea. Thus, stimulation to a plurality of locations of the subject 62 may be used and/or required to effectively treat all types of SA being experienced by the subject.

The process 180 may further include a determination of whether a sleep cycle of the subject 62 continues in block 214. The determination of whether a sleep cycle continues in block 214 may be manually inputted by the user 62, input by another user, or based upon selected sensor inputs such as a position sensor input.

If a sleep cycle is not continuing, a NO path 218 may be followed to end the process in block 220. Ending the process in block 220 may include placing the ID 30 into a sleep mode or rest mode to save battery life and reduce stimulation of the subject 62 during a wake period of the subject 62.

If it is determined that a sleep cycle is continuing in block 214, a YES path 224 may be followed. The YES path 224 may return the sensor signal in block 184 and/or receive the sensor signal in block 188. Accordingly, the ID 30 may be used in a continuous manner during a sleep cycle of the subject 62 to ensure appropriate therapy is provided to the subject 62.

Further, it is understood that the process 180 need not necessarily include sensing at the sensor in block 184. The process 180 may include only receiving the sensor signal in block 188 for process and analysis by the selected processor, such as the processor 50 of the ID 30. Accordingly, the process 180 may include a treatment of the subject 62 with the ID 30 for treating complex or inclusive sleep apnea of the subject 62 with the single ID 30 via the plurality of leads that are positioned at various and distinct locations within the subject 62. Further, the ID 30 may include various channels to allow for stimulation to each of the individual lead or lead contact rather than to all of the lead contacts simultaneously. It is understood, therefore, that the pattern of stimulation may be sequential and/or simultaneous and/or selective to the various lead contacts with reference to a selected treatment therapy that may be recalled from the memory 54.

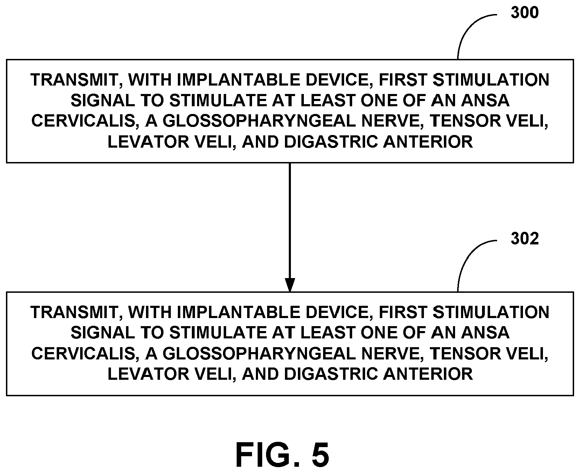

FIG. 5 is a flowchart illustrating an example method of sleep apnea treatment. In the example of FIG. 5, processor 50 may cause ID 30 to transmit a first stimulation signal to stimulate at least one of an ansa cervicalis, a glossopharyngeal nerve, tensor veli, levator veli, and digastric anterior of a patient (300). For example, the first stimulation signal may be configured to cause stimulation, contraction and/or traction of at least one of a sternohyoid, a sternothyroid, or an omohyoid muscle of the patient (e.g., through stimulation of the ansa cervicalis to stiffen neck muscles and prevent medial/lateral collapse). As another example, the first stimulation signal may be configured to cause a soft palate of the patient to tense and/or elevate (e.g., to open the airway and minimize sleep apnea).

Processor 50 may cause the same ID 30 to transmit a second stimulation signal to stimulate at least one of a hypoglossal nerve or a phrenic nerve of the patient (300). For instance, ID 30 may stimulate the hypoglossal nerve to prevent anterior/posterior collapse. As another example, ID 30 may stimulate the phrenic nerve to cause the diaphragm to move, and treat CSA. In some examples, ID 30 may be configured to stimulate both the hypoglossal nerve and the phrenic nerve. For example, the second stimulation signal may be configured to stimulate one of the hypoglossal nerve or the phrenic nerve, and processor 50 may be configured to cause ID 30 to transmit a third stimulation signal to a third lead to stimulate the other of the hypoglossal nerve or the phrenic nerve.

In some examples, to transmit the first stimulation signal and the second stimulation signal, processor 50 may be configured to cause ID 30 to at least one of alternate the first stimulation signal and the second stimulation signal, or simultaneously transmit the first stimulation signal and the second stimulation signal. In some examples, one or more sensors 61 may be configured to sense one or more parameters of the patient. In such examples, to transmit the first stimulation signal and the second stimulation signal, processor 50 may be configured to cause ID 30 to transmit the first stimulation signal and the second stimulation signal based on the one or more parameters. The one or more sensors 61 comprise a first sensor and a second sensor (e.g., sensors 61, 61', 61'', and 61'''). ID 30 may include the first sensor (e.g., sensor 61), and the second sensor may be external to the patient (e.g., sensor 61'). In some examples, one or more of sensors 61 may be within the patient but external to ID 60, such as sensors 61'' and 61'''.

As discussed above, therefore, the stimulation system 20, including the ID 30 may provide or direct stimulation to various positions that are spaced apart and/or are distinct form one another to provide different and/or varying therapies. The OSA therapy may include a first stimulation therapy with the leads 34a, b to the muscle groups at or near the tongue and/or a second therapy with the lead 34d to the AC. The CSA therapy may include a stimulation with the lead 34c to the phrenic nerve PN to activate the diaphragm. It is understood that the SA therapy may include all or one of these separate therapies in a selected manner and/or order.

The following describes some example techniques that may be implemented together or in any combination.

Example 1A. A system for selecting a parameter for activating one or more areas of muscle of a subject, comprising: a first lead and a second lead; a stimulation system configured to be implanted in the subject and having at least a first channel connected to the first lead and a second channel connected to the second lead; a memory system to store instructions to be executed to treat a sleep apnea of the subject; and a processor system configured to execute the stored instructions to: transmit a first stimulation signal to the first lead to provide a first sleep apnea therapy, and transmit a second stimulation signal to the second lead to treat a second sleep apnea therapy; and a power source configured to provide stimulation energy based on at least one of the first stimulation signal or the second stimulation signal; wherein the first sleep apnea differs from the second sleep apnea.

Example 2A. The system of example 1A, wherein the processor, the memory, and the channels are all included in the implantable stimulation system.

Example 3A. The system of example 2A, further comprising: a sensor configured to sense a parameter of the subject.

Example 4A. The system of example 3A, wherein the sensor is configured to send a sensor signal to the processor; wherein the processor compares the sensor signal to a stored parameter set to determine whether at least one of the first sleep apnea or the second sleep apnea is occurring in the subject.

Example 5A. The system of any of examples 1A-4A, wherein the first sleep apnea therapy includes stimulation of at least one of a first muscle group in an upper airway, a second muscle group in the upper airway, or a diaphragm; wherein the second sleep apnea therapy includes stimulation of at least one other of the first muscle group in the upper airway, the second muscle group in the upper airway, or the diaphragm.

Example 6A. A method of selecting a parameter for activating one or more areas of muscle of a subject, comprising: sensing a parameter of the subject; executing instructions with a processor to determine a first lead to send a first stimulation signal to provide a first therapy for a first sleep apnea and a second lead to send a second stimulation signal to provide a second therapy for a second sleep apnea; and stimulating the subject with the first lead and the second lead, wherein the second lead is to stimulate an ansa cervicalis of the subject.

Example 7A. The method of example 6A, wherein the stimulating the subject the second lead is configured to cause stimulation, contraction and/or traction of at least one of a sternohyoid, a sternothyroid, or a omohyoid muscle.

Example 8A. The method of any of examples 6A and 7A, further comprising at least one of alternating the first stimulation signal and the second stimulation signal; sequentially applying the first stimulation signal and the second stimulation signal; or applying only one of the first stimulation signal and the second stimulation signal.

Example 9A. The method of any of examples 6A-8A, wherein the first therapy includes stimulation of at least one of a first muscle group in an upper airway, a second muscle group in the upper airway, or a diaphragm; wherein the second therapy includes stimulation of at least one other of the first muscle group in the upper airway, the second muscle group in the upper airway, or the diaphragm.

Example 1B. A system for sleep apnea treatment, comprising: an implantable medical device (IMD) couplable to a first lead and a second lead, wherein the IMD comprises a processor and stimulation circuitry, and wherein the processor is configured to cause the stimulation circuitry of the IMD to: transmit a first stimulation signal to the first lead to stimulate at least one of an ansa cervicalis, a glossopharyngeal nerve, tensor veli, levator veli, and digastric anterior of a patient; and transmit a second stimulation signal to the second lead to stimulate at least one a hypoglossal nerve or a phrenic nerve of the patient.

Example 2B. The system of example 1B, wherein the first stimulation signal is configured to cause stimulation, contraction and/or traction of at least one of a sternohyoid, a sternothyroid, or an omohyoid muscle of the patient.

Example 3B. The system of example 1B, wherein the first stimulation signal is configured to cause a soft palate of the patient to tense and/or elevate.

Example 4B. The system of any of examples 1B-3B, wherein to transmit the first stimulation signal and the second stimulation signal, the processor is configured to cause the stimulation circuitry of the IMD to at least one of: alternate the first stimulation signal and the second stimulation signal; or simultaneously transmit the first stimulation signal and the second stimulation signal.

Example 5B. The system of any of examples 1B-4B, wherein the second stimulation signal is configured to stimulate one of the hypoglossal nerve or the phrenic nerve, and wherein the processor is configured to cause the stimulation circuitry of the IMD to transmit a third stimulation signal to a third lead to stimulate the other of the hypoglossal nerve or the phrenic nerve.

Example 6B. The system of any of examples 1B-5B, further comprising one or more sensors configured to sense one or more parameters of the patient, and wherein to transmit the first stimulation signal and the second stimulation signal, the processor is configured to cause the stimulation circuitry of the IMD to transmit the first stimulation signal and the second stimulation signal based on the one or more parameters.

Example 7B. The system of example 6B, wherein the one or more sensors comprise a first sensor and a second sensor, wherein the IMD includes the first sensor, and wherein the second sensor is external to the patient.

Example 8B. A method for sleep apnea treatment, the method comprising: transmitting, with an implantable medical device (IMD), a first stimulation signal to a first lead to stimulate at least one of an ansa cervicalis, a glossopharyngeal nerve, tensor veli, levator veli, and digastric anterior of a patient; and transmitting, with the same IMD, a second stimulation signal to the second lead to stimulate at least one a hypoglossal nerve or a phrenic nerve of the patient.

Example 9B. The method of example 8B, wherein the first stimulation signal is configured to cause stimulation, contraction and/or traction of at least one of a sternohyoid, a sternothyroid, or an omohyoid muscle of the patient.

Example 10B. The method of example 8B, wherein the first stimulation signal is configured to cause a soft palate of the patient to tense and/or elevate.

Example 11B. The method of any of examples 8B-10B, wherein transmitting the first stimulation signal and transmitting the second stimulation signal comprises: alternating transmitting the first stimulation signal and the second stimulation signal; or simultaneously transmitting the first stimulation signal and the second stimulation signal.

Example 12B. The method of any of examples 8B-11B, wherein the second stimulation signal is configured to stimulate one of the hypoglossal nerve or the phrenic nerve, the method further comprising transmitting, with the same IMD, a third stimulation signal to a third lead to stimulate the other of the hypoglossal nerve or the phrenic nerve.

Example 13B. The method of any of examples 8B-12B, further comprising receiving, from one or more sensors, one or more parameters of the patient, and wherein transmitting the first stimulation signal and transmitting the second stimulation signal comprises transmitting the first stimulation signal and transmitting the second stimulation signal based on the one or more parameters.

Example 14B. The method of example 13B, wherein the one or more sensors comprise a first sensor and a second sensor, wherein the IMD includes the first sensor, and wherein the second sensor is external to the patient.

Example 15B. A computer-readable storage medium storing instructions thereon that when executed cause one or more processors of an implantable medical device (IMD) to: cause the IMD to transmit a first stimulation signal to a first lead to stimulate at least one of an ansa cervicalis, a glossopharyngeal nerve, tensor veli, levator veli, and digastric anterior of a patient; and cause the same IMD to transmit a second stimulation signal to the second lead to stimulate at least one a hypoglossal nerve or a phrenic nerve of the patient.

Example 16B. The computer-readable storage medium of example 15B, wherein the first stimulation signal is configured to cause stimulation, contraction and/or traction of at least one of a sternohyoid, a sternothyroid, or an omohyoid muscle of the patient.

Example 17B. The computer-readable storage medium of example 15B, wherein the first stimulation signal is configured to cause a soft palate of the patient to tense and/or elevate.

Example 18B. The computer-readable storage medium of any of examples 15B-17B, wherein the instructions that cause the one or more processors to cause the IMD to transmit the first stimulation signal and transmit the second stimulation signal comprise instructions that cause the one or more processors to cause the IMD to: alternate transmitting the first stimulation signal and the second stimulation signal; or simultaneously transmit the first stimulation signal and the second stimulation signal.

Example 19B. The computer-readable storage medium of any of examples 15B-18B, wherein the second stimulation signal is configured to stimulate one of the hypoglossal nerve or the phrenic nerve, the instruction further comprising instructions that cause the one or more processors to cause the IMD to transmit a third stimulation signal to a third lead to stimulate the other of the hypoglossal nerve or the phrenic nerve.

Example 20B. The computer-readable storage medium of any of examples 15B-19B, further comprising instructions that cause the one or more processors to receive, from one or more sensors, one or more parameters of the patient, and wherein the instructions that cause the one or more processors to cause the IMD to transmit the first stimulation signal and transmit the second stimulation signal comprise instructions that cause the one or more processors to cause the IMD to transmit the first stimulation signal and transmit the second stimulation signal based on the one or more parameters.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium (e.g., memory module) and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors (e.g. processor module), such as one or more digital signal processors (DSPs), general purpose microprocessors, graphic processing units (GPUs), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Examples are provided so that this disclosure will be thorough, and will fully convey the scope to those who are skilled in the art. Numerous specific details are set forth such as examples of specific components, devices, and methods, to provide a thorough understanding of the present disclosure. It will be apparent to those skilled in the art that specific details need not be employed, that examples may be embodied in many different forms, and that neither should be construed to limit the scope of the disclosure. In some examples, well-known processes, well-known device structures, and well-known technologies are not described in detail.

The foregoing description of the examples has been provided for purposes of illustration and description. It is not intended to be exhaustive or to limit the disclosure. Individual elements or features of a particular embodiment are generally not limited to that particular example, but, where applicable, are interchangeable and can be used in a selected example, even if not specifically shown or described. The same may also be varied in many ways. Such variations are not to be regarded as a departure from the disclosure to the extent that they fall within the scope of the invention as defined by the claims.

## Claims

1. A system for sleep apnea treatment, comprising:
an implantable medical device, IMD, (30) couplable to a first lead (40) and a second lead (44), wherein the IMD comprises a processor (50) and stimulation circuitry (55), and wherein the processor is configured to cause the stimulation circuitry of the IMD to:
transmit a first stimulation signal to the first lead to stimulate at least one of an ansa cervicalis, a glossopharyngeal nerve, tensor veli, levator veli, and digastric anterior of a patient; and
transmit a second stimulation signal to the second lead to stimulate at least one a hypoglossal nerve or a phrenic nerve of the patient.

2. The system of claim 1, wherein the first stimulation signal is configured to cause stimulation, contraction and/or traction of at least one of a sternohyoid, a sternothyroid, or an omohyoid muscle of the patient.

3. The system of claim 1, wherein the first stimulation signal is configured to cause a soft palate of the patient to tense and/or elevate.

4. The system of claim 1, 2 or 3, wherein to transmit the first stimulation signal and the second stimulation signal, the processor is configured to cause the stimulation circuitry of the IMD to at least one of:
alternate the first stimulation signal and the second stimulation signal; or
simultaneously transmit the first stimulation signal and the second stimulation signal.

5. The system of any preceding claim, wherein the second stimulation signal is configured to stimulate one of the hypoglossal nerve or the phrenic nerve, and wherein the processor is configured to cause the stimulation circuitry of the IMD to transmit a third stimulation signal to a third lead to stimulate the other of the hypoglossal nerve or the phrenic nerve.

6. The system of any preceding claim, further comprising one or more sensors (61) configured to sense one or more parameters of the patient, and wherein to transmit the first stimulation signal and the second stimulation signal, the processor is configured to cause the stimulation circuitry of the IMD to transmit the first stimulation signal and the second stimulation signal based on the one or more parameters.

7. The system of claim 6, wherein the one or more sensors comprise a first sensor (61) and a second sensor (61'), wherein the IMD includes the first sensor, and wherein the second sensor is external to the patient.

8. A computer-readable storage medium storing instructions thereon that when executed cause one or more processors of an implantable medical device, IMD to:
cause the IMD to transmit a first stimulation signal to a first lead to stimulate at least one of an ansa cervicalis, a glossopharyngeal nerve, tensor veli, levator veli, and digastric anterior of a patient; and
cause the same IMD to transmit a second stimulation signal to the second lead to stimulate at least one a hypoglossal nerve or a phrenic nerve of the patient.

9. The computer-readable storage medium of claim 8, wherein the first stimulation signal is configured to cause stimulation, contraction and/or traction of at least one of a sternohyoid, a sternothyroid, or an omohyoid muscle of the patient.

10. The computer-readable storage medium of claim 8, wherein the first stimulation signal is configured to cause a soft palate of the patient to tense and/or elevate.

11. The computer-readable storage medium of claim 8, 9 or 10, wherein the instructions that cause the one or more processors to cause the IMD to transmit the first stimulation signal and transmit the second stimulation signal comprise instructions that cause the one or more processors to cause the IMD to:
alternate transmitting the first stimulation signal and the second stimulation signal; or
simultaneously transmit the first stimulation signal and the second stimulation signal.

12. The computer-readable storage medium of any of claims 8 to 11, wherein the second stimulation signal is configured to stimulate one of the hypoglossal nerve or the phrenic nerve, the instruction further comprising instructions that cause the one or more processors to cause the IMD to transmit a third stimulation signal to a third lead to stimulate the other of the hypoglossal nerve or the phrenic nerve.

13. The computer-readable storage medium of any of claims 8 to 12, further comprising instructions that cause the one or more processors to receive, from one or more sensors, one or more parameters of the patient, and wherein the instructions that cause the one or more processors to cause the IMD to transmit the first stimulation signal and transmit the second stimulation signal comprise instructions that cause the one or more processors to cause the IMD to transmit the first stimulation signal and transmit the second stimulation signal based on the one or more parameters.
